# EUROPEAN PATENT APPLICATION

(11) **EP 4 382 508 A1**
(43) Date of publication of application: **12.06.2024**
(21) Application number: 22212020.6
(22) Date of filing: 07.12.2022
(51) Int. Cl.: C07C 225/16

(54) **PROCESSES FOR MAKING ENDOCHIN-LIKE QUINOLONES FROM ALKYL 3-(3-ALKOXYPHENYL)AMINO)-2-(4-(4-ALKOXY)PHENOXY)PHENYL)BUT-2-ENOATE**

(71) Applicant: Intervet International B.V., 5831 AN Boxmeer (NL)
(72) Inventor: The designation of the inventor has not yet been filed
(74) Representative: Intervet International B.V.

(57) **Abstract**

A new scalable synthesis for the preparation of endochin-like quinolone compounds of Formula (I) 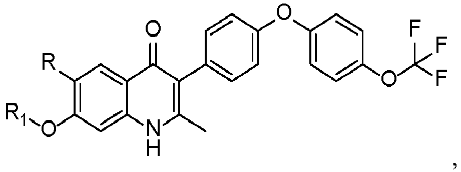 wherein R is Cl or F, and R₁ is C₁-C₂ alkyl, wherein the synthesis comprises is a process for preparing a compound of Formula 7 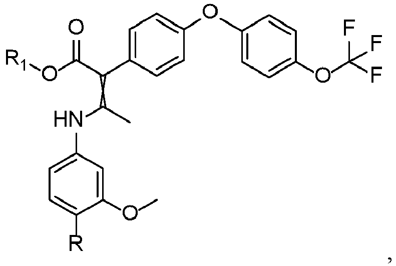 wherein R is Cl or F, preferably F and R₁ is C₁-C₂ alkyl, preferably Ci alkyl.

## Description

### Background

U.S. Patent No. 8,598,354 discloses endochin-like quinolone compounds having antiparasitic or anti-infectious activity including 6-fluoro-7-methoxy-2-methyl-3-(4- (4-(trifluoromethoxy)phenoxy)phenyl)quinolin-4(1H)-one (ELQ-316) (See Figure 4, sheet 23).

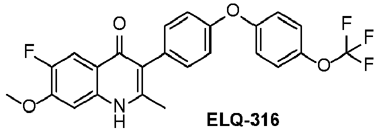

WO/2021/231335 discloses synthetic methods and novel intermediates in the preparation of 3-aryl endochin-like quinolone (ELQ) compounds including ELQ-316 that are amenable to industrial scaleup production.

Hammers, et al. describe the use of (diacetoxyiodo)benzene (also known as phenyliodine(III) diacetate, PhI(OAc)₂) and trimethyl orthoformate (TMOF) in methanol in the oxidative 1,2-aryl migration of a ketone to the corresponding α-methyl ester (Org. Biomol. Chem. 2021, 19, 2213-2223).

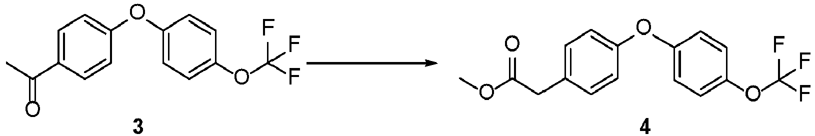

Attempts to perform the oxidative rearrangement of intermediate 3 into compound 4 either in the presence of iodic acid and sulfuric acid as described by Krishnacharya G. Akamanchi and coworkers in ARKIVOC 2011 (v) 67-75 or in the presence of iodine in trimethylorthoformate as described by Yamauchi et al. in J. Org. Chem. 1988, 53, 4858-4859; failed to produce the desired product 4.

Pou, et al. disclose synthetic routes to endochin-like quinolone compounds such as ELQ-300, ELQ-316, and other antiparasitic quinolones (Org. Process Res. Dev. 2021, 25, 1841-1852).

Specifically, Pou discloses another process for making a compound similar to compound 4 which involves the use of a metal (copper) at elevated temperature 160 °C. However, the reported yields remain moderate, 60% - 70%. Furthermore, Pou describes the use of acetic anhydride for introducing an acetyl group on compound 4 which leads to a mixture of compounds 5 and 5a. This mixture needs to be treated with an acid (para toluenesulfonic acid, TsOH) to convert 5a back to 5. This is an additional step and it is not easy to remove the TsOH from the product which had to be carried over in the next step.

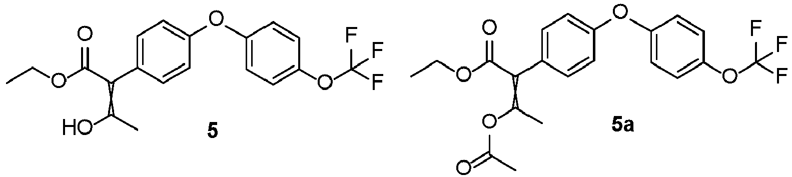

Vaswani et al. (Org. Lett. 2014, 16, 4114-4117) discloses the use of lithium bis(trimethylsilyl)amide (LiHMDS) and 1-(1H-imidazol-1-yl)ethanone at low temperature to acylate aryl α-methyl esters.

Atkins et al. (Org. Proc. Res. Dev. 1997, 1, 185-197) disclose ring closure reactions using POCl₃ at low temperature.

WO2021231335 discloses novel intermediates useful in the synthesis of endochin-like quinolone compounds.

### Summary of the Invention

An embodiment of the invention is a process for preparing a compound of Formula 7 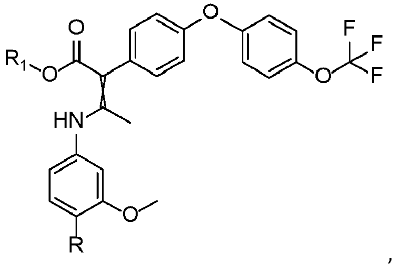
wherein R is Cl or F, preferably F and R₁ is C₁-C₂ alkyl, preferably C₁ alkyl,
comprising reacting a compound of Formula 5 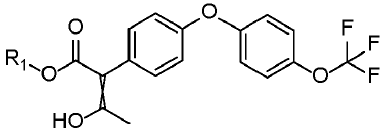
with a compound of Formula 6 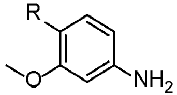
yield the compound of Formula 7.

### Detailed Descriptions

A new scalable synthesis for the preparation of endochin-like quinolone compounds of Formula (I) 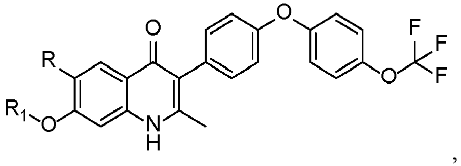 wherein R is Cl or F, and R₁ is C₁-C₂ alkyl which avoids the use of any metal catalysis (e.g. copper) and of harsh reaction conditions and allows the final compound to be isolated in very high purity (>99%) and in the absence of any trace of residual metal. Scheme 1 is representative where R is F and R₁ is methyl.

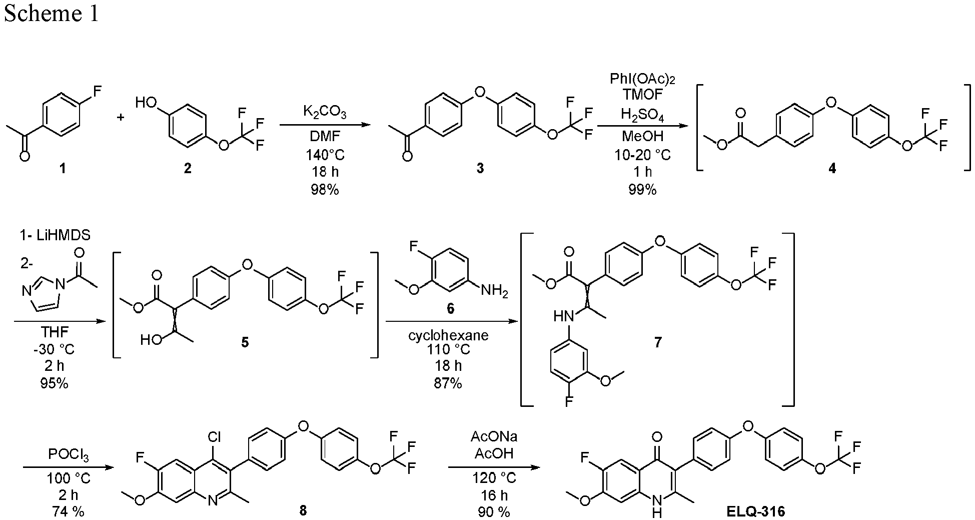

It has been discovered that the use of copper can be circumvented by reacting 4-fluoro acetophenone (Compound 1) with *p*-trifluoromethoxyphenol (Compound 2) in the presence of a base at 100 to 140°C. This temperature range is lower that other known similar reaction and is a significant advantage when the process is run at industrial scale. The resulting diarylether acetophenone (Compound 3) was obtained in nearly quantitative yield and then underwent an oxidative rearrangement to deliver the ester intermediate (Compound 4) in high yield and purity. This novel process allowed the isolation of compound (Compound 4) in the absence of any trace of residual metal. It is advantageous to avoid the use of copper in the synthesis of an active pharmaceutical ingredient (API). The process is applicable for large scale/industrial use.

It has also been discovered that the ester (Compound 4) can be conveniently converted into the keto-ester (Compound 5) in one step by using a base and acetylimidazole as alkylating agent and by carefully quenching the reaction mixture at low temperature (<10°C). This novel process allowed the isolation of the target keto-ester (Compound 5) in high yield and high purity. Under these new reaction conditions, the formation of the side product 5a, as reported in the literature, was not observed.

Further, it has been determined that a condensation / ring closing reaction performed in the presence of phosphorus oxychloride (POCl₃) under mild reaction conditions (80°C) allowed the isolation of a chloroquinoline intermediate (Compound 8) in high yield (~75%) and very high purity (>99%). This intermediate (Compound 8) was then converted into 6-fluoro-7-methoxy-2-methyl-3-(4- (4-(trifluoromethoxy)phenoxy)phenyl)quinolin-4(1H)-one in quantitative yield in the presence of sodium acetate in acetic acid. This compound was isolated in very high purity (>99%) and in the absence of any trace of residual metal.

AcOH is acetic acid.

AcONa is sodium acetate.

DMF is *N*,*N*-dimethyl formamide.

DMSO is dimethylsulfoxide.

LiHMDS is lithium bis(trimethylsilyl)amide or lithium hexamethyldisilazane.

LTMP is lithium tetramethylpiperidide.

LDA is lithium diisopropylamide.

MeOH is methanol.

NMP is *N*-methyl-2-pyrrolidone.

PCl₃ is phosphorus trichloride.

PCl₅ is phosphorous pentachloride.

PhI(OAc)₂ is (diacetoxyiodo)benzene or phenyliodonium diacetate.

POCl₃ is phosphorus oxychloride.

THF is tetrahydrofuran.

TMOF is trimethyl orthoformate.

Residual metals in pharmaceuticals that have no therapeutic value are considered contaminants and their levels are strictly controlled by the various regulatory agencies around the world (ICH guideline Q3D (R2) on elemental impurities). Substantially free means not present in numbers or quantities in excess of those that can be expected to result from and be consistent with normal handling and good cultural practices employed in the production and marketing of the commodity. With respect to residual metal, for example copper, in an active pharmaceutical ingredient (api) given parenterally, it means less than 300 ppm of the residual metal in the api composition.

An embodiment of the invention is a process for preparing a compound of Formula 4 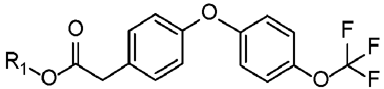
wherein R₁ is C₁-C₂ alkyl, preferably C₁ alkyl,
comprising reacting a compound of Formula 3 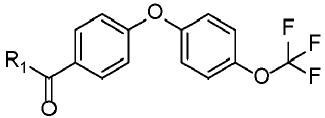
with phenyliodonium diacetate (PhI(OAc)₂) and an orthoformate to yield the compound of Formula 4.

In another embodiment of the invention, the orthoformate is trimethyl- or triethyl- orthoformate, preferably trimethyl orthoformate (TMOF).

In another embodiment of the invention, the reaction is conducted in an alcohol.

In another embodiment of the invention, the alcohol is methanol, ethanol, n-propanol or isopropanol, preferably methanol.

In another embodiment of the invention, the compound of Formula 3 is produced by reacting a compound of Formula 1 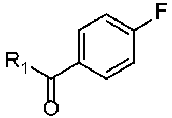
with a compound of Formula 2 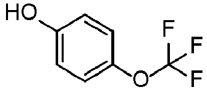
to yield the compound of Formula 3, wherein the temperature of the reaction is between about 100°C to about 170°C, between about 100°C to about 150°C, between about 125°C to about 150°C, between about 130°C to about 150°C, between about 100°C to about 145°C, between about 125°C to about 145°C, between about 100°C to about 140°C, preferably about 135°C to about 145°C.

In another embodiment the temperature is about 140°C.

In another embodiment of the invention, the reaction further comprises a base.

In another embodiment of the invention, the base is potassium carbonate, sodium carbonate or cesium carbonate, preferably potassium carbonate.

In another embodiment of the invention, the base is sodium carbonate.

In another embodiment of the invention, the base is cesium carbonate.

In another embodiment of the invention, the reaction further comprises a solvent.

In another embodiment of the invention, the solvent is dimethyl formamide (DMF), *N*-methyl-2-pyrrolidone (NMP) or dimethylsulfoxide (DMSO), preferably DMF.

In another embodiment of the invention, the solvent is NMP.

In another embodiment of the invention, the solvent is DMSO.

In an alternative embodiment of the invention, the process further comprises reacting the compound of Formula 4 to produce the compound of Formula (I) 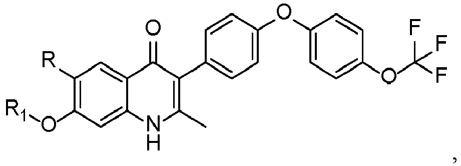 wherein R is Cl or F, preferably F and R₁ is C₁-C₂ alkyl, preferably C₁ alkyl.

In another embodiment of the invention, R is Cl and R₁ is C₁ alkyl.

In another embodiment of the invention, R is F and R₁ is C₁ alkyl.

In another embodiment of the invention, the compound of Formula (I) is substantially free of residual metal.

Another embodiment of the invention is a compound of Formula 4

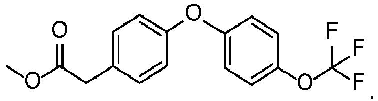

An embodiment of the invention is a process for preparing a compound of Formula 5 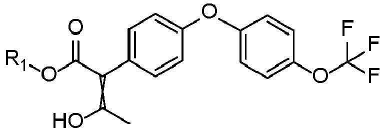
wherein R₁ is C₁-C₂ alkyl, preferably C₁ alkyl,
comprising reacting a compound of Formula 4 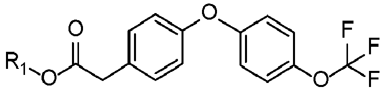
with a base and then an alkylating agent to produce the compound of Formula 5.

In another embodiment of the invention, the base is lithium bis(trimethylsilyl)amide (LiHMDS), lithium tetramethylpiperidide (LTMP) or lithium diisopropylamide (LDA), preferably LiHMDS.

In another embodiment of the invention, the base is LTMP.

In another embodiment of the invention, the base is LDA.

In another embodiment of the invention, the alkylating agent is acetylimidazole.

In another embodiment of the invention, the process is quenched at a temperature less than about 0°C to about 20°C, preferably less than about 10°C.

In another embodiment of the invention, the process is quenched at a temperature of between about 0°C to about 20°C, or between about 0°C to about 10°C or between about 10°C to about 20°C.

In an alternative embodiment of the invention, the process further comprises reacting the compound of Formula 5 to produce the compound of Formula (I) 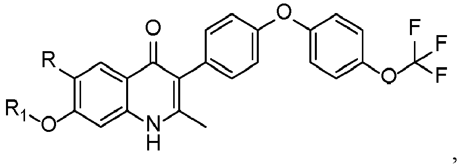 wherein R is Cl or F, preferably F and R₁ is C₁-C₂ alkyl, preferably C₁ alkyl.

In another embodiment of the invention, R is Cl and R₁ is C₁ alkyl.

Another embodiment of the invention is a compound of Formula 5

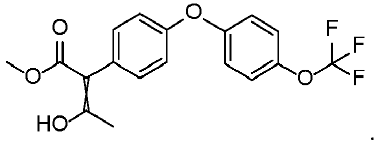

An embodiment of the invention is a process for preparing a compound of Formula 7 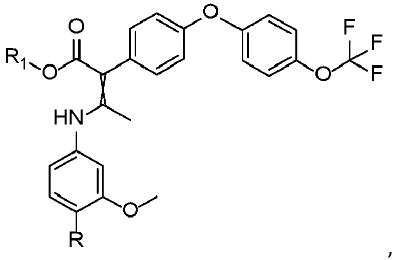
wherein R is Cl or F, preferably F and R₁ is C₁-C₂ alkyl, preferably C₁ alkyl,
comprising reacting a compound of Formula 5 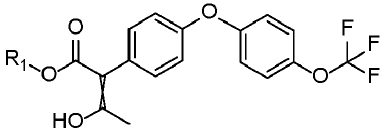
with a compound of Formula 6 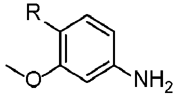
yield the compound of Formula 7.

In another embodiment of the invention, a solvent is used in the process.

In another embodiment of the invention, the solvent is an aprotic apolar solvent such as cyclohexane, toluene, heptane, xylene, or mixtures thereof, preferably cyclohexane.

In another embodiment of the invention, the solvent is heptane.

In another embodiment of the invention, the solvent is toluene.

In another embodiment of the invention, the temperature of the process is between about 50 °C and about 140°C, between about 60 °C and about 130 °C, about 70 °C and about 120°C, about 80 °C and about 120 °C, about 85 °C and about 110 °C, preferably about 90 °C and about 110 °C.

In another embodiment the temperature is about 110°C.

In an alternative embodiment of the invention, the process further comprises reacting the compound of Formula 7 with phosphorus oxychloride (POCl₃), phosphorus trichloride (PCl₃), or phosphorous pentachloride (PCl₅), preferably POCl₃, to yield a compound of Formula 8 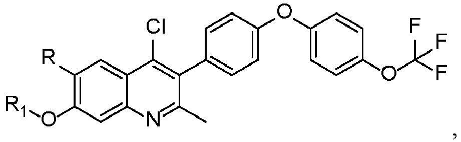 wherein R is Cl or F, preferably F and R₁ is C₁-C₂ alkyl, preferably C₁ alkyl.

In another embodiment of the invention, the temperature of the process is between about 50 °C and about 140 °C, between about 60 °C and about 130 °C, about 70 °C and about 120 °C, about 80 °C and about 120 °C, about 85 °C and about 110 °C, preferably about 90 °C and about 110 °C.

In another embodiment the temperature is about 100°C.

In an alternative embodiment of the invention, the process further comprises reacting the compound of Formula 8 to produce the compound of Formula (I) 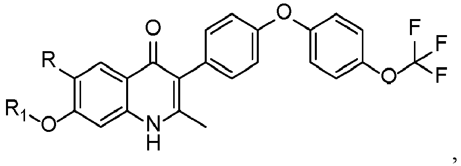 wherein R is Cl or F, preferably F and R₁ is C₁-C₂ alkyl, preferably C₁ alkyl.

In another embodiment of the invention, R is Cl and R₁ is C₁ alkyl.

In another embodiment, the process to form the compound of Formula (I) from the compound of Formula 8 is carried out *in situ* from the process to form the compound of Formula 8 from the compound of Formula 7.

Another embodiment of the invention is a compound of Formula 7

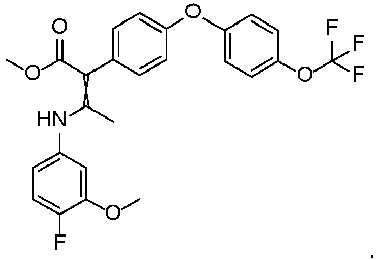

An embodiment of the invention is a process for preparing a compound of Formula (I) 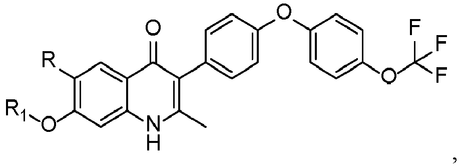
comprising reacting a compound of Formula 8 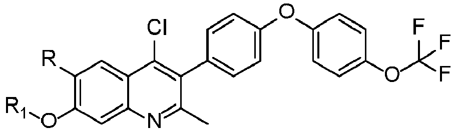
with an acetate salt in an acid to yield the compound of Formula (I), wherein R is Cl or F, preferably F and R₁ is C₁-C₂ alkyl, preferably C₁ alkyl.

In another embodiment of the invention, R is Cl and R₁ is C₁ alkyl.

In another embodiment of the invention, the acetate salt is potassium acetate or sodium acetate, preferably sodium acetate.

In another embodiment of the invention, the acetate salt is potassium acetate.

In another embodiment of the invention, the acid is acetic acid or a C₁-C₄ aliphatic acid, preferably acetic acid.

In another embodiment of the invention, the acid is a C₁-C₄ aliphatic acid.

In another embodiment of the invention, the process to form the compound of Formula (I) from the compound of Formula 8 is carried out *in situ* from a process to form the compound of Formula 8 from the compound of Formula 7

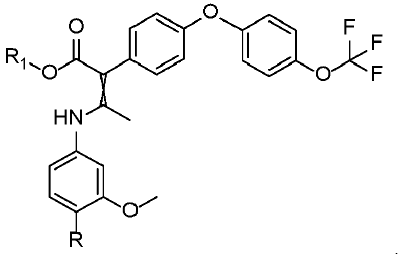

An alternative embodiment of the invention is a to produce the compound of Formula (I) 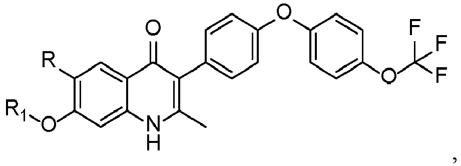
wherein R is Cl or F, preferably F and R₁ is C₁-C₂ alkyl, preferably C₁ alkyl,
comprising
   i) reacting a compound of Formula 1 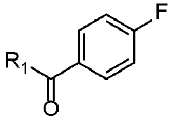
      with a compound of Formula 2 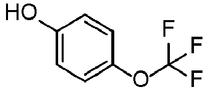
      to yield the compound of Formula 3, 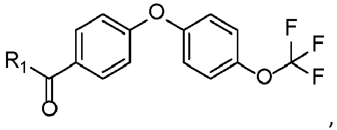
      wherein the temperature of the reaction is between about 100°C to about 170°C;
   ii) reacting a compound of Formula 3 with phenyliodonium diacetate (PhI(OAc)₂) and an orthoformate to produce the compound of Formula 4 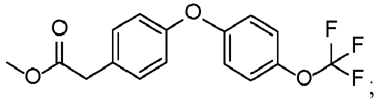
   iii) reacting a compound of Formula 4 with a base and then an alkylating agent to produce the compound of Formula 5 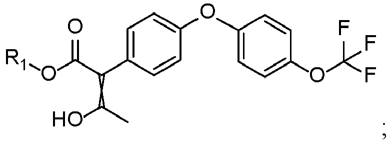 and
   iv) reacting the compound of Formula 5 with a compound of formula 6 to produce the compound of Formula (I).

An alternative embodiment of the invention is a to produce the compound of Formula (I) 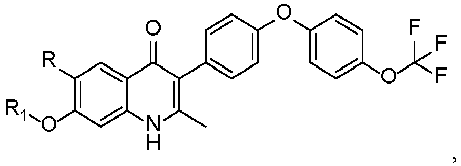
wherein R is Cl or F, preferably F and R₁ is C₁-C₂ alkyl, preferably C₁ alkyl,
comprising
   i) reacting a compound of Formula 1 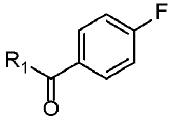
      with a compound of Formula 2 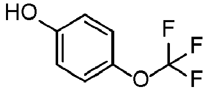
      to yield the compound of Formula 3, 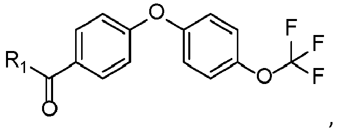
      wherein the temperature of the reaction is between about 100°C to about 170°C;
   ii) reacting a compound of Formula 3 with phenyliodonium diacetate (PhI(OAc)₂) and an orthoformate to produce the compound of Formula 4 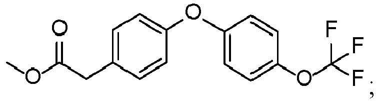
   iii) reacting a compound of Formula 4 with a base and then an alkylating agent to produce the compound of Formula 5 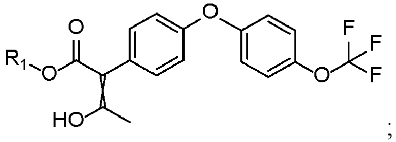
   iv) reacting the compound of Formula 5 with a compound of Formula 6 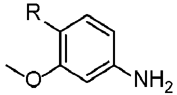
      yield the compound of Formula 7 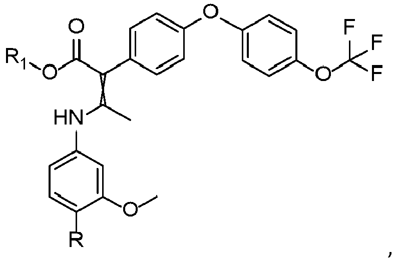
      wherein R is Cl or F, preferably F and R₁ is C₁-C₂ alkyl, preferably C₁ alkyl,;
   v) reacting the compound of Formula 7 to produce a compound of Formula 8 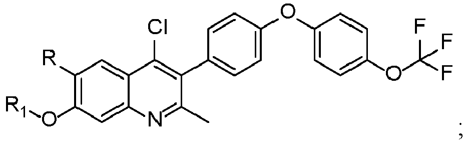 and
   vi) reacting the compound of Formula 8 to produce the compound of Formula (I).

An alternative embodiment of the invention is a to produce the compound of Formula (I) 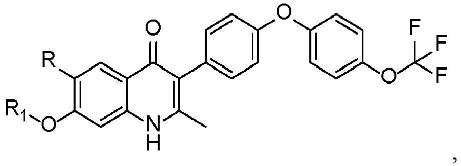
wherein R is Cl or F, preferably F and R₁ is C₁-C₂ alkyl, preferably C₁ alkyl,
comprising
   i) reacting a compound of Formula 1 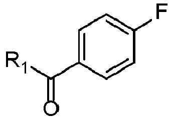
      with a compound of Formula 2 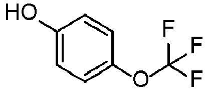
      to yield the compound of Formula 3, 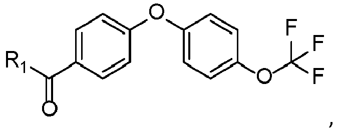
      wherein the temperature of the reaction is between about 100°C to about 170°C;
   ii) reacting a compound of Formula 3 with phenyliodonium diacetate (PhI(OAc)₂) and an orthoformate to produce the compound of Formula 4 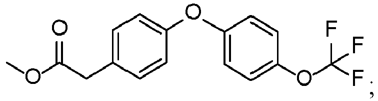
   iii) reacting a compound of Formula 4 with a base and then an alkylating agent to produce the compound of Formula 5 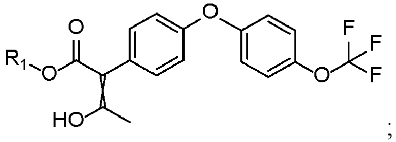
   iv) reacting the compound of Formula 5 with a compound of Formula 6 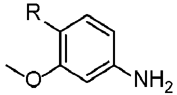
      yield the compound of Formula 7 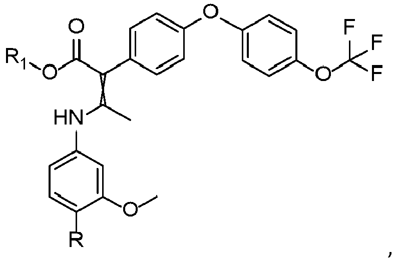
      wherein R is Cl or F, preferably F and R₁ is C₁-C₂ alkyl, preferably C₁ alkyl,;
   v) reacting the compound of Formula 7 to produce a compound of Formula 8 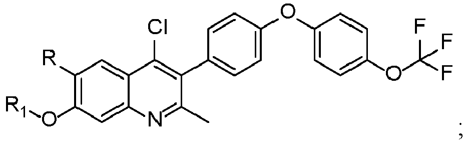
   vi) reacting the compound of Formula 8 with an acetate salt in an acid to produce the compound of Formula (I).

### EXAMPLES

### HPLC methods:

### Method A

Agilent Technologies UHPLC/MSD 6130 B Series 1290 composed of binary pump G7120A included degasser, Well plate sampler G4226A, Column oven G1316B, Diode array detector G4212A, Mass detector G6130B Quadrupole LC/MS with ESI-source.
Column: Waters XP, 2.1 × 50mm Xbridge BEH C18 2.5 µ, T = 40 °C;
Eluents: A: acetonitrile with 0.05 % (vol./vol.) formic acid.
B: water with 0.05 % formic acid (vol./vol.);
Flow: 0.8 mL/min;
Gradient: from 2 to 100 % eluent A 1.2 min, 0.5 min 100 % eluent A;
Run time: 2.2 min;
Detection: ESI/MS, positive and negative ions scan: 100-1000 m/z;
UV at 254 and 210 nm.

### Example 1: Preparation of methyl 2-(4-(4-(trifluoromethoxy)phenoxy)phenyl) acetate (Compound 4)

### Step 1: Preparation of 1-(4-(4-(trifluoromethoxy)phenoxy) phenyl)ethan-1-one (Compound 3)

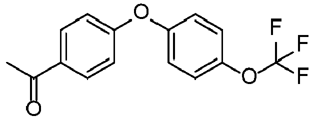

Potassium carbonate (39.6 g, 287 mmol) was added to a solution of 1-(4-fluorophenyl)ethan-1-one (17.70 mL, 143 mmol) and 4-(trifluoromethoxy)phenol (20.84 mL, 158 mmol) in *N,N-*dimethylformamide (400 mL). The resulting mixture was heated to 140 °C and was stirred at this temperature for 16 h. The reaction mixture was then allowed to reach room temperature, was diluted with water (1 L) and was extracted with ethyl acetate (2 × 500 mL). The combined organic layers were washed with aqueous 1N hydrochloric acid (500 mL), were dried and concentrated under reduced pressure to afford a brown oil. The obtained oil was dissolved in dichloromethane (1 L), was filtered over a short pad of silica gel and the filtrate was concentrated under reduced pressure to afford the desired product as a brown oil (43.6 g, 140 mmol) which was directly engaged in step 2.

### NMR

¹H NMR (300 MHz, Methanol-d₃) δ (ppm): 8.04 - 8.01 (m, 2H); 7.35 (d, J = 8.4 Hz, 2H); 7.20 - 7.15 (m, 2H); 7.08 - 7.04 (m, 2H); 2.58 (s, 3H)

¹³C NMR (75 MHz, Methanol-d₃) δ (ppm): 197.7; 161.6; 154.4; 145.4; 132.2; 130.6; 122.8; 121.0; 117.2; 25.2

UPLC/MS (A_BEHC18): Rt = 1.23 min.

### Step 2: Preparation of methyl 2-(4-(4-(trifluoromethoxy)phenoxy)phenyl)acetate (Compound 4)

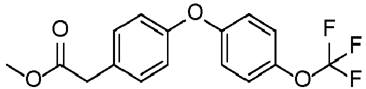

The crude product obtained from step 1 (43.6 g, 140 mmol) was dissolved in methanol (430 mL), trimethoxymethane (127 mL, 1161 mmol) was added and the temperature of the resulting mixture was adjusted to 10 °C. Concentrated sulfuric acid (64.8 mL, 1161 mL) and phenyliodine(III) diacetate (50.1 g, 152 mmol) were sequentially added while holding the temperature of the reaction mixture between 10 to 16 °C. After completion of the additions, the reaction mixture was allowed to reach room temperature and was stirred for 90 min. The temperature was then adjusted to 5 °C and water (500 mL) was added. The volume of the resulting mixture was reduced under reduced pressure (distillated volume ~ 70 mL), water (1 L) was added and the pH was set to 7 by the addition of sodium hydrogen carbonate. The aqueous solution was extracted with dichloromethane (2 × 300 mL) and the combined organic layers were dried and concentrated under reduced pressure to afford the desired product (71.3 g, 63 wt.%) in the presence of iodobenzene (37 wt.%).

¹H NMR (600 MHz, Dichloromethane-d₂) δ (ppm): 7.33 - 7.28 (m, 2H); 7.24 (dd, *J* = 0.7, 9.0 Hz, 2H); 7.07 - 7.04 (m, 2H); 7.03 - 7.00 (m, 2H); 3.72 (s, 3H); 3.65 (s, 1H)

¹³C NMR (75 MHz, Methanol-d₃) δ (ppm): 171.9; 155.9; 144.4; 130.8; 129.8; 122.6; 121.4; 119.6; 118.9; 51.9; 40.1

UPLC/MS (A_BEHC18): Rt = 1.26 min; m/z 325.

### Example 2: Preparation of 4-chloro-6-fluoro-7-methoxy-2-methyl-3-(4-(4-(trifluoromethoxy)phenoxy)phenyl)quinoline (Compound 8)

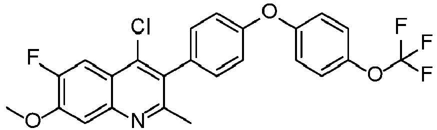

### Step 1: Preparation of methyl 3-oxo-2-(4-(4-(trifluoromethoxy)phenoxy)phenyl) butanoate (Compound 5)

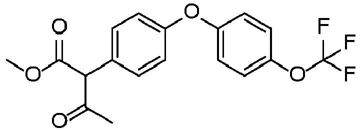

Methyl 2-(4-(4-(trifluoromethoxy)phenoxy)phenyl)acetate (20.0 g, 63 wt.%, 38.6 mmol) obtained from step 2 of example 1 and 1-(1*H*-imidazol-1-yl)ethan-1-one (5.1 g, 46.3 mmol) were placed under nitrogen atmosphere and were dissolved in a mixture of dry tetrahydrofuran (200 mL) and dry *N,N*-dimethylacetamide (20 mL). The temperature of the resulting solution was set to - 30 °C and a 1M solution of lithium bis(trimethylsilyl)amide in tetrahydrofuran (93 mL, 93 mmol) was added while maintaining the temperature between -31 to -28 °C. After completion of the addition (about 20 min), the resulting mixture was stirred for 1 h between -30 to -25 °C. Cooling was stopped and saturated aqueous ammonium chloride (100 mL) was slowly added to the reaction mixture which resulted in an increase in temperature to -1 °C. The mixture was allowed to reach room temperature and was stirred for 30 min. After dilution with water (200 mL), the mixture was extracted with ethyl acetate (200 mL). The organic layer was sequentially washed with aqueous 2N hydrochloric acid (200 mL), with brine (200 mL) and was concentrated under reduced pressure to afford the desired product as a brown oil (18.5 g) which was directly engaged in the next step.

UPLC/MS (A_BEHC18): Rt = 1.16, 1.23, 1.26 and 1.36 min; m/z 367.

### Step 2: Preparation of methyl 3-((4-fluoro-3-methoxyphenyl)amino)-2-(4-(4-(trifluoromethoxy)phenoxy)phenyl)but-2-enoate (Compound 7)

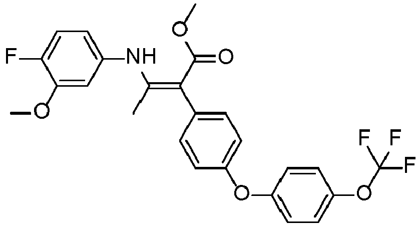

The crude product isolated from step 1 (18.5 g, 38.7 mmol) was dissolved in cyclohexane (150 mL), 4-fluoro-3-methoxyaniline (5.46 g, 38.7 mmol) was added and the resulting mixture was refluxed (at about 110 °C) for 17 h while removing the formed water. After 17 h reaction time, the reaction mixture was concentrated under reduced pressure to afford the desired product as a brown oil (22.9 g) which was directly engaged in the next step.

### Step 3: Preparation of 4-chloro-6-fluoro-7-methoxy-2-methyl-3-(4-(4-(trifluoromethoxy)phenoxy)phenyl)quinoline (Compound 8)

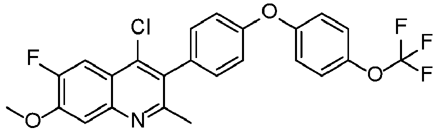

To the crude product isolated from step 2 (51.2 g, 72.9 mmol) was added phosphoryl trichloride (50 mL, 536 mmol) and the resulting mixture was heated at 100 °C to react for 2 h. The temperature of the reaction mixture was lowered to 5 °C, dichloromethane (50 mL) was added and water (500 mL) was slowly added under vigorous stirring while maintaining the temperature below 15 °C. The reaction mixture was extracted with dichloromethane (300 mL), the organic layer was collected, was washed sequentially with aqueous saturated sodium hydrogen carbonate (250 mL) and with water (250 mL) and was concentrated under reduced pressure. The obtained residue was triturated with ethanol (200 mL) at 45 °C for 20min. The formed suspension was filtered, the wet cake was rinsed with ethanol (150 mL) and the obtained solid was dried under reduced pressure at 40 °C to afford the desired product as a white solid (29.3 g, 60.7 mmol).

### NMR

¹H NMR (300 MHz, Dimethysulfoxide-d₆) δ (ppm): 7.89 (d, *J* = 6.0 Hz, 1H); 7.66 (d, *J* = 4.2 Hz, 1H); 7.45 (d, *J* = 4.2 Hz, 2H); 7.41 - 7.39 (m, 2H); 7.24 - 7.23 (m, 2H); 7.20 - 7.18 (m, 2H); 4.04 (s, 3H); 2.40 (s, 3H)

¹³C NMR (75 MHz, Dimethysulfoxide-d₆) δ (ppm): 158.2; 156.7; 155.6; 153.3; 151.6; 151.1; 151.0; 145.7; 144.5; 139.5; 139.4; 132.1; 123.6; 121.4; 120.9; 119.8; 119.4; 119.2; 110.6; 109.1; 108.9; 57.0; 25.4

¹⁹F NMR (282 MHz, Dimethysulfoxide-d₆) δ (ppm): -57.1; -131.1

UPLC/MS (A_BEHC18): Rt = 1.45 min; m/z 325.

### Example 3: Preparation of 6-fluoro-7-methoxy-2-methyl-3-(4-(4-(trifluoromethoxy)phenoxy)phenyl)quinolin-4(1H)-one (Compound ELQ-316)

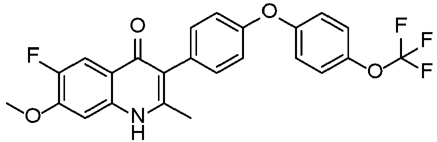

4-chloro-6-fluoro-7-methoxy-2-methyl-3-(4-(4-(trifluoromethoxy)phenoxy)phenyl) quinoline (28 g, 58.0 mmol) was dissolved in acetic acid (300 mL), sodium acetate was added (9.52 g, 116 mmol) and the resulting mixture was reacted at 120 °C for 16 h. The obtained suspension was allowed to reach room temperature, was diluted with water (300 mL) and was filtered. The wet cake was sequentially rinsed with water (250 mL) and with acetone (2 × 125 mL) and the obtained solid was dried under reduced pressure at 40 °C to deliver the desired product as a white solid (23.1 g, 50 mmol).

### NMR

¹H NMR (300 MHz, *N,N*-Dimethylformamide-d₇) δ (ppm): 7.83 (d, *J* = 11.5 Hz, 1H); 7.49 (d, *J* = 8.8 Hz, 1H); 7.42 - 7.40 (m, 2H); 7.27 - 7.24 (m, 2H); 7.22 (d, *J* = 6.9 Hz, 1H); 7.15 - 7.13 (m, 2H); 4.05 (s, 3H); 2.36 (s, 3H)

¹³C NMR (75 MHz, *N,N*-Dimethylformamide-d₇) δ (ppm): 174.4; 156.5; 155.3; 151.5; 151.4; 150.3; 148.7; 146.5; 144.2; 137.6; 132.3; 123.2; 121.5; 119.9; 118.5; 110.4; 110.3; 56.1; 18.6

¹⁹F NMR (282 MHz, *N,N*-Dimethylformamide-d₇) δ (ppm): -57.2; -140.7

UPLC/MS (A_BEHC18): Rt = 1.13 min; m/z 460.

### Example 4: Preparation of 4,6-dichloro-7-methoxy-2-methyl-3-(4-(4-(trifluoromethoxy)phenoxy)phenyl)quinoline

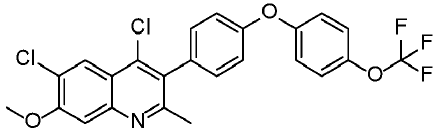

### Step 1: Preparation of methyl 3-((4-chloro-3-methoxyphenyl)amino)-2-(4-(4-(trifluoromethoxy)phenoxy)phenyl)but-2-enoate

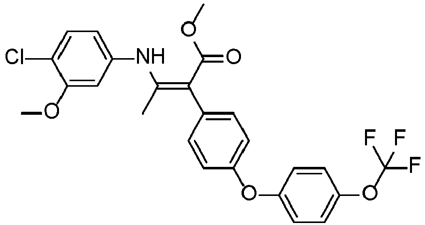

The crude product isolated from step 1 of example 2 (2.0 g, 5.43 mmol) was dissolved in cyclohexane (15 mL), 4-chloro-3-methoxyaniline (0.87 g, 5.43 mmol) was added and the resulting mixture was refluxed (at about 110 °C) for 24 h while removing the formed water. Heating was stopped and the reaction mixture was concentrated under reduced pressure to afford the desired product as a brown oil (2.9 g) which was directly engaged in the next step.

### Step 2: Preparation of 4,6-dichloro-7-methoxy-2-methyl-3-(4-(4-(trifluoromethoxy)phenoxy)phenyl)quinoline

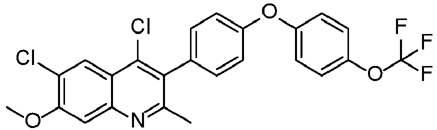

To the crude product isolated from step 1 (2.9 g) was added phosphoryl trichloride (10.6 mL, 114 mmol) and the resulting mixture was heated at 100 °C to react for 135 min. The temperature of the reaction mixture was lowered to 5 °C, dichloromethane (50 mL) was added and water (100 mL) was slowly added under vigorous stirring while maintaining the temperature below 15 °C. The reaction mixture was extracted with dichloromethane (25 mL), the organic layer was collected, was washed sequentially with aqueous saturated sodium hydrogen carbonate (50 mL) and with water (50 mL) and was concentrated under reduced pressure. The obtained residue was triturated with ethanol (10 mL) at 45 °C for 20min. The formed suspension was filtered, the wet cake was rinsed with ethanol (10 mL) and the obtained solid was dried under reduced pressure at 40 °C to afford the desired product as a white solid (1.3 g, 2.49 mmol).

### NMR

¹H NMR (300 MHz, Dimethysulfoxide-d₆) δ (ppm): 8.18 (s, 1H); 7.63 (s, 1H); 7.47- 7.45 (m, 2H); 7.42 - 7.40 (m, 2H); 7.24 - 7.23 (m, 2H); 7.20 - 7.19 (m, 2H); 4.05 (s, 3H); 2.41 (s, 3H) ¹³C NMR (75 MHz, Dimethysulfoxide-d₆) δ (ppm): 159.4; 156.7; 156.4; 155.6; 147.7; 144.5; 139.1; 132.5; 132.1; 131.8; 124.9; 124.6; 123.6; 121.4; 119.8; 119.2; 109.4; 57.3; 25.6

¹⁹F NMR (282 MHz, Dimethysulfoxide-d₆) δ (ppm): -57.1

UPLC/MS (A_BEHC18): Rt = 1.50 min; m/z 494.

### Example 5: Preparation of 6-chloro-7-methoxy-2-methyl-3-(4-(4-(trifluoromethoxy)phenoxy)phenyl)quinolin-4(1H)-one

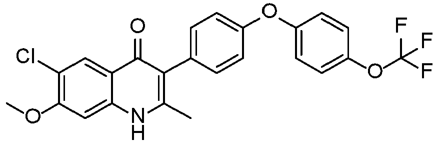

4,6-Dichloro-7-methoxy-2-methyl-3-(4-(4-(trifluoromethoxy)phenoxy)phenyl) quinoline (1 g, 2.02 mmol) was dissolved in acetic acid (10 mL), sodium acetate (0.33 g, 4.05 mmol) was added and the resulting mixture was reacted at 120 °C for 17 h. The obtained suspension was allowed to reach room temperature, was diluted with water (15 mL) and was filtered. The wet cake was rinsed with water (10 mL) and the obtained solid was dried under reduced pressure at 40 °C to deliver the desired product as a white solid (0.9 g, 1.74 mmol).

UPLC/MS (A_BEHC18): Rt = 1.17 min; m/z 476.

## Claims

1. A process for preparing a compound of Formula 7 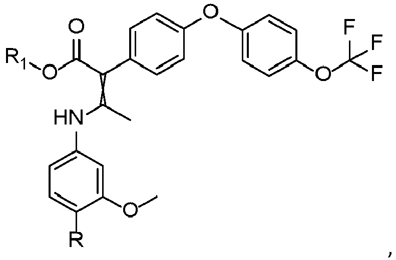
wherein R is Cl or F, preferably F and R₁ is C₁-C₂ alkyl, preferably C₁ alkyl,
comprising reacting a compound of Formula 5 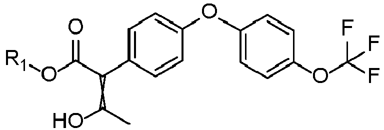
with a compound of Formula 6 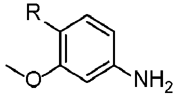
yield the compound of Formula 7.

2. The process of claim 1, further comprising reacting the compound of Formula 7 with phosphorus oxychloride (POCl₃), phosphorus trichloride (PCl₃), or phosphorous pentachloride (PCl₅), preferably POCl₃, to yield a compound of Formula 8 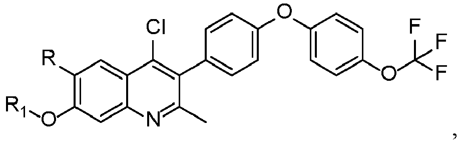 wherein R is Cl or F, preferably F and R₁ is C₁-C₂ alkyl, preferably C₁ alkyl.

3. The process of claim 2, wherein a solvent is used in the process.

4. The process of claim 3, wherein the solvent is an aprotic apolar solvent such as cyclohexane, toluene, heptane, xylene, or mixtures thereof, preferably cyclohexane.

5. The process of any one of claims 1-4, wherein the temperature of the process is between about 80 °C and about 120 °C, preferably about 85 °C and about 115 °C.

6. The process of any one of claims 2 to 5, further comprising reacting the compound of Formula 8 to produce the compound of Formula (I) 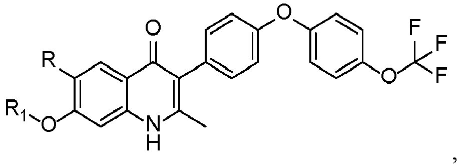 wherein R is Cl or F, preferably F and R₁ is C₁-C₂ alkyl, preferably C₁ alkyl.

7. The process of claim 6, wherein the temperature of the process is between about 80 °C and about 120 °C, preferably about 90 °C and about 110 °C.

8. The process of any one of claims 6 or 7, wherein the process to form the compound of Formula (I) from the compound of Formula 8 is carried out *in situ* from the process to form the compound of Formula 8 from the compound of Formula 7.

9. A compound of Formula 7
